# EUROPÄISCHE PATENTSCHRIFT

(11) **EP 1 500 697 B1**
(45) Veröffentlichungstag und Bekanntmachung des Hinweises auf die Patenterteilung: **21.10.2015**
(21) Anmeldenummer: 04017315.5
(22) Anmeldetag: 22.07.2004
(51) Int. Cl.: G01N 33/50

(54) **Bioartifizielles, primär vaskularisiertes Testgewebe für pharmakologische Testverfahren, Verfahren zu dessen Herstellung und Testverfahren unter dessen Verwendung sowie Bioreaktor zur Durchführung der Verfahren**
Bioartificial, primarily vascularized tissue for pharmaceutical tests, method for its production, use of it in a bioreactor.
Tissu bioartificiel vascularisé de façon primaire pour utilisation dans un procédé de test pharmacologique, procédé de production dudit tissu, procédé de test utilisant ledit tissu et bioréacteur pour réaliser le procédé.

(30) Priorität: 24.07.2003 DE 10333863
(43) Veröffentlichungstag der Anmeldung: 26.01.2005
(73) Patentinhaber: Fraunhofer-Gesellschaft zur Förderung der angewandten Forschung e.V., 80686 München (DE)
(72) Erfinder: Walles, Thorsten, Dr., 71063 Sindelfingen (DE)
(74) Vertreter: Bremer, Ulrich

(56) Entgegenhaltungen:
- WO-A-02/14480
- WO-A-02/35992
- WO-A-02/064179
- WO-A-03/043675
- US-A1- 2001 039 047
- SCHMIDT C E ET AL: "ACELLULAR VASCULAR TISSUES: NATURAL BIOMATERIALS FOR TISSUE REPAIR AND TISSUE ENGINEERING" BIOMATERIALS, ELSEVIER SCIENCE PUBLISHERS BV., BARKING, GB, Bd. 21, November 2000 (2000-11), Seiten 2215-2231, XP000985305 ISSN: 0142-9612
- KOUNIAVSKY GUENADI ET AL: "Stromal extracellular matrix reduces chemotherapy-induced apoptosis in colon cancer cell lines" CLINICAL AND EXPERIMENTAL METASTASIS, Bd. 19, Nr. 1, 2002, Seiten 55-60, XP002371163 ISSN: 0262-0898
- MERTSCHING H ET AL: "Engineering of a vascularized scaffold for artificial tissue and organ generation" BIOMATERIALS, ELSEVIER SCIENCE PUBLISHERS BV., BARKING, GB, Bd. 26, Nr. 33, November 2005 (2005-11), Seiten 6610-6617, XP004989117 ISSN: 0142-9612
- CHUNG YOUNG ET AL: "Human embryonic stem cell lines generated without embryo destruction", CELL STEM CELL, ELSEVIER, CELL PRESS, vol. 2, no. 2, 7 February 2008 (2008-02-07), pages 113-117, XP002604696, ISSN: 1934-5909, DOI: 10.1016/J.STEM.2007.12.013

## Beschreibung

Die Erfindung betrifft ein Verfahren zum Testen eines Pharmakons oder einer strahlentherapeutischen Behandlung.

Im Rahmen von Pharmakaentwicklungen spielt der Einfluss der verwendeten Chemikalien und neu entwickelten Pharmaka auf den Zellzyklus aller Zellen eine zentrale Rolle, da deren Proliferation, Syntheseraten oder angiogenetische Einflüsse gezielt verstärkt oder gestoppt werden sollen. Zur Überprüfung solch neu entwickelter Pharmaka werden vor der klinischen Phase Tierexperimente durchgeführt, die die Effektivität, Toxizität sowie die Nebenwirkungen des entsprechenden neuen Produktes dokumentieren. Hierzu werden meist Nacktmaus-Systeme oder Großtiere wie Schwein, Hund oder Schaf eingesetzt. Die in diesen Tiersystemen erhaltenen Ergebnisse sind nicht direkt auf den Menschen übertragbar. Trotz der hohen genetischen Homologie zwischen z. B. Mensch und Maus von z. B. 92 % können die von einem Mausmodell erhaltenen Ergebnisse nicht auf das humane System transferiert werden.

Es wurden daher in den letzten Jahren verstärkt Zellkultursysteme entwickelt, in denen humane Zellen ex vivo mit den entsprechenden Wirkstoffen in Kontakt kommen. Der Einfluss auf deren Zellzyklus kann so untersucht werden. Die humanen Zellkulturen werden im Wesentlichen zweidimensional in einem Nährstoffmedium mit den verschiedenen Pharmaka in Kontakt gebracht und die Auswirkungen in Abhängigkeit verschiedener Parameter untersucht. Es zeigt sich jedoch, dass die gefundenen Veränderungen der Zellkulturen nicht zwangsläufig auf den Wirkstoff zurückzuführen sind und somit wiederum auch derartige Ergebnisse nicht direkt der Auswirkung eines Pharmakons auf den menschlichen Körper gleichzusetzen sind. In den Zellkulturen finden hierbei insbesondere Dedifferenzierungsprozesse in Abhängigkeit der entsprechenden Umgebung (Mikroenviroment) statt, die zu Veränderungen führen, die von Veränderungen aufgrund der zugeführten Pharmaka schwer zu differenzieren sind.

Die US 2001/039047 A1 beschreibt ein Verfahren zum Herstellen eines dreidimensionalen, bioartifiziellen Gewebes mit lebenden Zellen in oder auf einer Matrix, wobei die Zellen und die Matrix in einem Gewebe oder einem Vorläufer-Gewebe kultiviert werden können. Weiterhin sind ein vaskularisiertes Gewebe aus biologischem Material, das durch ein derartiges Verfahren hergestellt wird, und ein Reaktor zu dessen Herstellung vorgesehen.

Die WO 02/35992 A2 beschreibt die Ausbildung künstlicher Gewebe, die eine endotheliale Oberfläche erfordern, wie z.B. Blutgefäße und Herzklappen.

Die WO 03/043675 A beschreibt die Erzeugung bzw. Rekonstruktion künstlicher weiblicher Reproduktionsorgane, wie z.B. Uterus, Vagina und Zervix. Hierbei werden Zellpopulationen aus geeignetem Zellmaterial perfundiert.

Die WO 02/064179 A2 beschreibt eine pioartifzielle, primär vaskularisierte Gewebematrix und ein entsprechendes Gewebe sowie Verfahren zu deren Herstellung und Verwendung.

Der Erfindung liegt die Aufgabe zugrunde, gegenüber bekannten Testverfahren Verbesserungen zu schaffen und insbesondere Testverfahren von Pharmaka und strahlentherapeutischen Behandlungen zu ermöglichen, die möglichst weitgehend auf den menschlichen Körper übertragen werden können.

Diese Aufgabe wird gelöst durch ein Verfahren nach Anspruch 1 sowie ein Verfahren nach Anspruch 5. Die Unteransprüche beschreiben bevorzugte Weiterbildungen.

Der Erfindung liegt die Erkenntnis zu Grunde, dass fehlende Übereinstimmungen zwischen ex vivo - Zellkultursystemen aus humanen Zellen und dem menschlichen Körper insbesondere auf die zweidimensionale Umgebung derartiger Zellkultursysteme zurückzuführen sind. Diese neuen Zellen werden ex vivo in der zweidimensionalen oder quasi zweidimensionalen Umgebung vermehrt und können aufgrund ihres zweidimensionalen Mikroenviroment Dedifferenzierungsprozessen unterworfen werden. Derartige Dedifferenzierungsprozesse können in vivo insbesondere durch das Zellkulturmedium oder extrazelluläre Matrixkomponenten verhindert werden.

Um derartige Dedifferenzierungsprozesse zu vermeiden und die in einem nativen dreidimensionalen Gewebeverband stattfindenden Wechselwirkungen nachzubilden, werden erfindungsgemäß zur Durchführung der Testverfahren Konzepte aus der Implantationstechnik, des so genannten Tissue Engineerings, genutzt. Endothelzellen, humane Primär- oder Tumorzellen und eine natürliche vaskularisierte Trägerstruktur, d. h. extrazellulärer Matrix ECM, werden als Ersatz für einen nativen dreidimensionalen Gewebeverband herangezogen. Eine Testsubstanz oder eine physikalische Behandlung kann in vitro auf die Zellen einwirken, so dass der Einfluss eines Wirkstoffs oder der physikalischen Behandlung auf Zellzyklus, Proliferation und Syntheseraten in einem natürlichen bzw. quasi natürlichen Mikroenviroment untersucht werden kann. So können beispielsweise Veränderungen in der Entstehungsrate neuer Blutgefäße und Variationen im Expressionsmuster endothelialer Adhäsionsmoleküle beurteilt werden. Zusätzlich besteht die Möglichkeit, spezielle Zell-Zell und Zell-Matrix Interaktionen auf mRNA und Proteinebene zu untersuchen. Gefundene Veränderungen können mit der eingesetzten Pharmaka-Konzentration und der Applikationsdauer korreliert werden, woraufhin nach der entsprechenden Auswertung die entsprechenden Parameter verändert werden können.

Erfindungsgemäß wird hiermit zunächst aus einem biologischen Ausgangsgewebe durch Entzellularisieren eine bioartifizielle, vaskularisierte, biologische Gewebematrix aus im Wesentlichen Kollagen gebildet. Vorteilhafterweise wird das Gewebe hierzu vollständig dezellularisiert; falls autologes Gewebe genommen wird, kann z. B. auch eine nur teilweise Dezellularisierung vorgenommen werden. Eine derartige Herstellung einer Gewebematrix ist für sich genommen bereits aus dem Bereich der Implantatherstellung bzw. Tissue Engineering, z. B. der WO 02/064179 A2 bekannt. Hierbei kann z. B. ein xenogenes Gewebe aus z. B. einem Schwein, ein autologes Gewebe des Patienten selbst oder ein allogenes Gewebe eines anderen humanen Patienten verwendet werden, wobei das Gewebe mindestens einen Gefäßast, vorzugsweise einen arteriellen und einen venösen Gefäßast aufweist. Es werden Endothelzellen oder Stammzellen oder Progeniturzellen zugefügt, die in der primär vaskularisierten Gewebematrix ein Gefäßnetz ausbilden. Ergänzend werden die für die späteren Testverfahren relevanten humanen Zellen, insbesondere Tumorzellen zugeführt. Weiterhin können insbesondere auch Leberzellen zugeführt werden. Leberzellen haben hierbei die für die Testverfahren vorteilhafte Eigenschaft, die zugeführten Medikamente und Abbauprodukte zu verstoffwechseln.

Erfindungsgemäß können weiterhin auch humane Stammoder Vorläuferzellen zugeführt werden, da diese sich in dem Gewebe entsprechend den Kulturbedingungen zu verschiedenen Zelltypen ausbilden können, z.B. Muskel- oder Knorpelzellen. Hierdurch wird erfindungsgemäß ein frisches Gewebe erzeugt, das sich insbesondere aufgrund seiner höheren Teilungs- und Regenerationsrate sowie aufgrund fehlender vorheriger Einflüsse gut für das nachfolgende Testverfahren eignet. Hierbei können adulte Stammzellen verwendet werden.

Erfindungsgemäß wird somit ein ex vivo - "Organsystem" nachgebildet. Es hat sich hierbei gezeigt, dass zur Ausbildung eines realistischen Systems eine gute Perfusion bereits der Gewebematrix während der Besiedlung mit den humanen Zellen erforderlich ist. Hierbei zeigte sich insbesondere auch eine gute und realistische Ausbildung des Testgewebes bei einer gepulsten Zuführung des Nährstoffmedium. Als Nährstoffmedium kann bereits bei der Besiedlung mit den Humanzellen Blut, insbesondere autologes Blut zugeführt werden. Weiterhin können auch bereits während der Besiedlung Pharmaka dem Nährstoffmedium, insbesondere dem Blut zugeführt werden.

Das Testgewebe ermöglicht insbesondere zwei Anwendungsbereiche:
Zum einen können im Bereich der Grundlagenforschung hierdurch pharmakokinetische Prozesse und strahlungstherapeutische Prozesse in humanen primären Zellen oder Tumoren untersucht werden. Hieraus können neue Therapeutika entwickelt werden.

Zum anderen kann für individuelle Patienten oder einzelne Patientengruppen in klinischen Anwendungen die Wirksamkeit spezifischer, vorzugsweise pharmakologischer Therapiekonzepte, insbesondere auch kombinierter pharmakologischer und strahlentherapeutischer Therapiekonzepte überprüft und weiterentwickelt werden. Insbesondere bei derartigen klinischen Anwendungen ist die Zuführung von patienteneigenem Blut während der Besiedlung und/oder auch während des Testsverfahrens vorteilhaft. Es kann somit bis auf ggf. eine nicht autologe, im Wesentlichen aus Kollagen bestehende Gewebematrix durch Besiedlung mit autologen primären Zellen oder Tumorzellen und Versorgung mit autologem

Blut ein sehr realistisches, bioartifizielles, biologisches ex vivo Quasi-Organsystem gebildet werden.

Erfindungsgemäß kann vorteilhafterweise der Stoffwechsel kontrolliert und ggf. eingestellt werden. Insbesondere hierbei ergeben sich deutliche Vorteile gegenüber bekannten zweidimensionalen Zellkultursystemen. Es können insbesondere Messreihen in Abhängigkeit von Pharmakakonzentrationen, Zufuhrbedingungen, ggf. weiteren strahlentherapeutischen Behandlungen und Versorgung mit weiteren Nährstoffen untersucht werden.

Die Perfusion des Quasi-Organsystems findet vorzugsweise in einem erfindungsgemäßen Bioreaktor statt. Das Perfusionssystem verfügt über mindestens einen, vorzugsweise fünf Adapter-/anschlussysteme für Perfusionsleitungen. Das Lumen des Organsystems wird über einen Zu- und einen Ablauf mit Kulturlösung perfundiert. Die Gefäßäste der biologischen Matrix werden gesondert an eine Perfusionseinheit angeschlossen und von dieser perfundiert. Die Perfusion ist unter laminaren und pulsatilen Perfusionsbedingungen möglich. Unter der Perfusion und während der Kulturbedingungen können jederzeit Proben aus dem Perfusat gewonnen werden und Pharmaka, Zelllösungen oder andere Wirkstoffe hinzugegeben werden.

Erfindungsgemäß kann vorteilhafterweise die Herstellung des Testgewebes in dem gleichen Bioreaktor durchgeführt werden wie das nachfolgende Testverfahren; hierbei können die Anschlüsse des arteriellen und venösen Gefäßastes des Testgewebes an den Zuleitungsstutzen des Bioreaktors verbleiben, so dass nachteilhafte Einwirkungen durch eine zwischenzeitliche Entnahme des gesamten Testgewebes weitgehend vermieden werden können.

Die Erfindung wird im folgenden anhand eines Beispiels erläutert.

Das Beispiel betrifft die Herstellung und Kultivierung von humanem 3D-Tumorgewebe in vitro aus Tumorbiopsien. Einem Tumorpatienten mit einem seltenen oder therapierefraktären Malignom wird eine Gewebebiopsie des Tumorareals sowie 200 ml Blut entnommen. Die Blutprobe wird 1:1 mit PBS verdünnt und jeweils 10 ml davon auf 20 ml Bicoll-Lösung aufgegeben. Die mononuklearen Zellen werden durch eine Zentrifugation für 20 min bei 300g ohne Bremse isoliert, und zweimal mit PBS gewaschen.

Die gewonnenen mononukleären Zellen dienen zur Re-Endothelialisierung einer aus azellularisiertem Schweinedarm gefertigten biologischen Trägerstruktur mit erhaltenem Gefäßbett mit einem arteriellen und einem venösen Gefäßast. Die durch die Azellularisierung erhaltene Gewebematrix wird in einem Inkubationsbehältnis, das auch als Bioreaktor bezeichnet wird, fixiert und mit einem Nährstoffmedium und Endothelzellen oder Stamm- und Progeniturzellen perfundiert und bildet so ein funktionelles intramurales Kapillar- und Gefäßnetz, das sich dreidimensional durch das Gewebe erstreckt. Hierbei kann zur Perfusion insbesondere Blut, vorzugsweise patienteneigenes, d. h. autologes Blut verwendet werden.

Das Tumorgewebe wird nach steriler Entnahme in eine Petrischale gegeben, mit 2 ml RPMI befeuchtet und in kleine Stücke geschnitten. Nekrotisches und Fettgewebe werden entfernt. Die kleingeschnittenen Fragmente des Tumorgewebes werden mit 400 IU Collagnease/ml Hank's Solution für 4 Stunden bei 37° C inkubiert. Abhängig von der Konsistenz des Tumorgewebes kann die Collagenasekonzentration bis auf 1300-1500 IU/ml Hank's und die Inkubationszeit bis auf 15-24 Stunden erhöht werden. Zur Vermeidung von Zellagregatbildungen sollte dann allerdings 2 IU/ml Heparin der Hank's Lösung zugesetzt werden. Nach der Inkubationsphase wird die Lösung durch ein Zellsieb filtriert, um den Zelldebris abzutrennen. Die erhaltene Zellsuspension wird für 10 min bei 200g zentrifugiert und somit die Tumorzellen sedimentiert. Der Überstand wird verworfen und das Zellpellet zweimal mit 20 ml frischem RPMI Medium gewaschen. Die Zellzahl wird in einer Neugebaurzählkammer bestimmt, und die Tumorzellen werden in einer Zelldichte von 1x10⁵ auf die Gewebematrix aufgebracht und unter Perfusion kultiviert.

Unter den gegebenen Kulturbedingungen entwickelt sich humanes dreidimensional vaskularisiertes Tumorgewebe ex vivo. Nach zweiwöchiger Kultivierungsphase werden die bioartifiziellen Tumorgewebe mit einem Pharmakon inkubiert. Hierzu wird das Pharmakon dem Nährstoffmedium, vorzugsweise dem patienteneigenen Blut, über die Gefäßäste zugeführt.

Der Effekt der Pharmakaexposition lässt sich mittels histologischen (z. B. HE, Pentachrom), immunhistochemischen, proteinbiochemischen (z. B. Western-Blot) oder molekularbiologischen (z. B. RT-PCR) Untersuchungen nachweisen.

In der beigefügten Zeichnung ist ein Beispiel eines erfindungsgemäßen Bioreaktors zur Durchführung des Herstellungs- und Testverfahrens gezeigt.

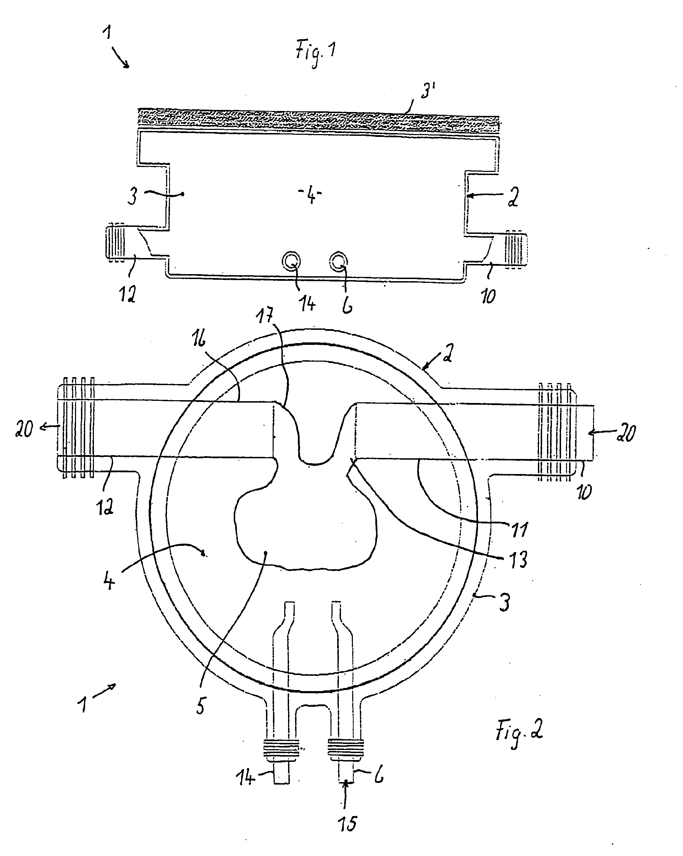

Es zeigen
- Fig. 1: eine Seitenansicht des erfindungsgemäßen Bioreaktors und
- Fig. 2: eine Draufsicht auf den Bioreaktor nach Fig. 1 mit abgenommenem Deckel.

Gleiche oder einander entsprechende Bauteile in den Figuren der Zeichnung sind mit den gleichen Bezugszeichen versehen.

Ein Bioreaktor 1 weist einen Behälter 2 aus Glas mit einem Behälterunterteil 3 und einem Deckel 3' auf, die eine Gewebekulturkammer 4 zur Aufnahme einer Gewebematrix bzw. des hieraus gebildeten Testgewebes 5 umgeben. In die Gewebekulturkammer 4 laufen extern angeschlossene Leitungen 6, 10, 12, 14, die eine am Boden oder unteren Bereich der Seitenwand einmündende Kulturmedium-Zuleitung 6 zur Zuführung eines Kulturmediums 15, eine arterielle Zuleitung 10 mit einem Stutzen 11 zum Anschluss eines arteriellen Gefäßastes 13 des Testgewebes 5 und eine venöse Abführleitung 12 mit einem Stutzen 16 zum Anschluss eines venösen Gefäßastes 17 des Testgewebes 5 umfassen.

Durch die arterielle Zuleitung 10 wird als Nährstoffmedium 20 vorzugsweise Blut des Patienten zugeführt, dem ein Pharmakon - das auch ein Gemisch mehrerer Wirkstoffe sein kann - zugesetzt wird. Hierbei wird das Nährstoffmedium laminar oder pulsierend zugeführt.

Die Temperatur der zu- und abgeführten Lösungen sowie auch des Kulturmediums 15 in der Kammer 4 werden geregelt. Weiterhin wird der pH-Wert des Kulturmediums 15 in der Kammer 4 gemessen und kann geregelt werden.

Durch die weitere Zuleitung 14 können Proben aus der Kammer 14 entnommen werden und ggf. Wirkstoffe zugeführt werden. Hierbei kann z.B. die Auswirkung eines in das Gewebe eines Patienten gespritzten Zellgiftes zur Behandlung eines Tumors überprüft werden, indem dieses über die Zuleitung 14 zugeführt wird.

## Patentansprüche

1. Verfahren zum Testen eines Pharmakons oder einer strahlentherapeutischen Behandlung, mit mindestens folgenden Schritten:
- Herstellen eines bioartifiziellen, primär vaskularisierten Testgewebes mit folgenden Schritten: zumindest teilweises Dezellularisieren eines autologen, allogenen oder xenogenen Ausgangsgewebes mit wenigstens einem Gefäßast, zu einer bioartifiziellen, primär vaskularisierten, biologischen Gewebematrix mit wenigstens einer Matrix eines Gefäßasts und einem Kapillarnetz, Erhalt der Funktionsfähigkeit des Gefäßastes , Perfusion der Gewebematrix mit einem ersten Nährstoffmedium über die Matrix des wenigstens einen Gefäßastes; Auskleiden des Gefäßastes und des Kapillarnetzes mit Endothel-, Stamm- oder Vorläuferzellen und Besiedeln der Gewebematrix mit humanen Tumor-, Stamm-, Vorläufer- oder Leberzellen,
- Zuführen des Pharmakons in einem zweiten Nährstoffmedium, das dem mindestens einen Gefäßast zugeführt wird, oder strahlentherapeutische Behandlung des Testgewebes, und
- Untersuchen des Testgewebes.

2. Verfahren nach Anspruch 2, **dadurch gekennzeichnet, dass** beim Schritt des Herstellens das erste Nährstoffmedium Blut, vorzugsweise autologes Blut, ist

3. Verfahren nach Anspruch 1 oder 2, **dadurch gekennzeichnet, dass** beim Schritt des Herstellens das Ausgangsgewebe vollständig dezellularisiert wird.

4. Verfahren nach einem der vorherigen Ansprüche, **dadurch gekennzeichnet, dass** beim Schritt des Herstellens die Gewebematrix während der Besiedelung pulsierend perfundiert wird.

5. Verfahren zum Testen eines Pharmakons oder einer strahlentherapeutischen Behandlung, mit mindestens folgenden Schritten:
- Herstellen eines bioartifiziellen, primär vaskularisierten Testgewebes, aufweisend eine dezellularisierte, primär vaskularisierte, biologische Gewebematrix mit wenigstens einem Gefäßast, wobei die Gewebematrix mit humanen Tumor-, Stamm-, Vorläufer- oder Leberzellen besiedelt ist, und das Gewebe ein Kapillarund Gefäßnetz aufweist,
- Zuführen des Pharmakons in einem zweiten Nährstoffmedium, das dem mindestens einen Gefäßast zugeführt wird, oder strahlentherapeutische Behandlung des Testgewebes, und
- Untersuchen des Testgewebes.

6. Verfahren nach Anspruch 5, **dadurch gekennzeichnet, dass** beim Schritt des Herstellens die Gewebematrix wenigstens eine Matrix eines arteriellen Gefäßastes und wenigstens eine Matrix eines venösen Gefäßastes aufweist.

7. Verfahren nach Anspruch 5 oder 6, **dadurch gekennzeichnet, dass** beim Schritt des Herstellens der wenigstens eine Gefäßast mit einem in der Gewebematrix ausgebildeten intramuralen Kapillarnetz verbunden ist.

8. Verfahren nach einem der Ansprüche 5 bis 7, **dadurch gekennzeichnet, dass** beim Schritt des Herstellens das Testgewebe durchblutet wird.

9. Verfahren nach einem der vorherigen Ansprüche, **dadurch gekennzeichnet, dass** beim Schritt des Pharmakon-Zuführens das dem mindestens einen Gefäßast zugeführte zweite Nährstoffmedium Blut, vorzugsweise autologes Blut ist.

10. Verfahren nach einem der vorherigen Ansprüche, **dadurch gekennzeichnet, dass** beim Schritt des PharmakonZuführens das zweite Nährstoffmedium gepulst zugeführt wird.

11. Verfahren nach einem der vorherigen Ansprüche, **dadurch gekennzeichnet, dass** die strahlentherapeutische Behandlung und die Zuführung des Pharmakons gleichzeitig und/oder zeitlich getrennt durchgeführt werden.

12. Verfahren nach einem der vorherigen Ansprüche, **dadurch gekennzeichnet, dass** beim Schritt des Untersuchens Zellzyklus, Proliferation und Syntheseraten des Testgewebes untersucht werden.

13. Verfahren nach einem der vorherigen Ansprüche, **dadurch gekennzeichnet, dass** beim Schritt des Untersuchens Veränderungen in der Entstehungsrate neuer Blutgefäße und Variationen im Expressionsmuster von Adhäsionsmolekülen untersucht werden.

14. Verfahren nach einem der vorherigen Ansprüche, **dadurch gekennzeichnet, dass** beim Schritt des Untersuchens ZellZell- und Zell-Matrix-Interaktionen auf RNA- und Proteinebene untersucht werden.

15. Verfahren nach einem der vorherigen Ansprüche, **dadurch gekennzeichnet, dass** beim Schritt des Untersuchens ein Stoffwechsel des Testgewebes durch Überprüfung des zugeführten und abgeführten Nährstoffmediums untersucht wird.

## Claims

1. Method for testing a pharmacon or a radiotherapeutic treatment, comprising at least the following steps:
- preparation of a bioartificial, primarily vascularised test tissue by means of the following steps: at least partial decellularisation of an autologous, allogenic or xenogenic starting tissue with at least one vessel ramus to give a bioartificial, primarily vascularised, biological tissue matrix with at least one matrix of a vessel ramus and a capillary network, preservation of the functionality of the vessel ramus, perfusion of the tissue matrix with a first nutrient medium via the matrix of the at least one vessel ramus; lining of the vessel ramus and of the capillary network with endothelial cells, stem cells or precursor cells, and population of the tissue matrix with human tumour cells, stem cells, precursor cells or hepatocytes,
- delivery of the pharmacon in a second nutrient medium, which is delivered to the at least one vessel ramus, or radiotherapeutic treatment of the test tissue, and
- examination of the test tissue.

2. Method according to claim 1, **characterised in that** in the preparation step the first nutrient medium is blood, preferably autologous blood.

3. Method according to claim 1 or 2, **characterised in that** in the preparation step the starting tissue is decellularised completely.

4. Method according to any one of the preceding claims, **characterised in that** in the preparation step the tissue matrix is perfused in a pulsating manner during the population.

5. Method for testing a pharmacon or a radiotherapeutic treatment, comprising at least the following steps:
- preparation of a bioartificial, primarily vascularised test tissue having a decellularised, primarily vascularised, biological tissue matrix with at least one vessel ramus, wherein the tissue matrix is populated with human tumour cells, stem cells, precursor cells or hepatocytes, and the tissue has a capillary and vessel network,
- delivery of the pharmacon in a second nutrient medium, which is delivered to the at least one vessel ramus, or radiotherapeutic treatment of the test tissue, and
- examination of the test tissue.

6. Method according to claim 5, **characterised in that** in the preparation step the tissue matrix has at least one matrix of an arterial vessel ramus and at least one matrix of a venous vessel ramus.

7. Method according to claim 5 or 6, **characterised in that** in the preparation step the at least one vessel ramus is connected to an intramural capillary network formed in the tissue matrix.

8. Method according to any one of claims 5 to 7, **characterised in that** in the preparation step the test tissue is supplied with blood.

9. Method according to any one of the preceding claims, **characterised in that** in the step of delivery of the pharmacon the second nutrient medium delivered to the at least one vessel ramus is blood, preferably autologous blood.

10. Method according to any one of the preceding claims, **characterised in that** in the step of delivery of the pharmacon the second nutrient medium is delivered in a pulsed manner.

11. Method according to any one of the preceding claims, **characterised in that** the radiotherapeutic treatment and the delivery of the pharmacon are carried out simultaneously and/or chronologically separately.

12. Method according to any one of the preceding claims, **characterised in that** in the examination step cell cycle, proliferation and synthesis rates of the test tissue are examined.

13. Method according to any one of the preceding claims, **characterised in that** in the examination step changes in the rate of generation of new blood vessels and variations in the expression pattern of adhesion molecules are examined.

14. Method according to any one of the preceding claims, **characterised in that** in the examination step cell-cell and cell-matrix interactions at RNA and protein level are examined.

15. Method according to any one of the preceding claims, **characterised in that** in the examination step a metabolism of the test tissue is examined by checking the delivered and discharged nutrient medium.

## Revendications

1. Procédé d'essai d'un agent pharmaceutique ou d'un traitement par radiothérapie comportant au moins les étapes suivantes :
- fabrication d'un tissu échantillon bioartificiel à vascularisation primaire comportant les étapes suivantes :
décellularisation au moins partielle d'un tissu de départ autologue, allogénique ou xénogénique avec au moins une branche vasculaire pour obtenir une matrice tissulaire biologique à vascularisation primaire avec au moins une matrice d'une branche vasculaire et un réseau capillaire, maintien de la fonctionnalité de la branche vasculaire,
perfusion de la matrice tissulaire avec un premier milieu nutritif via la matrice de l'au moins une branche vasculaire, revêtement de la branche vasculaire et du réseau capillaire de cellules endothéliales, souches ou progénitrices et colonisation de la matrice tissulaire par des cellules tumorales, souches, progénitrices ou hépatiques humaines,
- amenée de l'agent pharmaceutique dans un second milieu nutritif, qui est amené à l'au moins une branche vasculaire ou traitement par radiothérapie du tissu échantillon, et
- examen du tissu échantillon.

2. Procédé suivant la revendication 2, **caractérisé en ce qu'à** l'étape de fabrication, le premier milieu nutritif est du sang, de préférence du sang autologue.

3. Procédé suivant la revendication 1 ou 2, **caractérisé en ce qu'à** l'étape de fabrication, le tissu de départ est entièrement décellularisé.

4. Procédé suivant une des revendications précédentes, **caractérisé en ce qu**'à l'étape de fabrication, la matrice tissulaire est perfusée de façon pulsée pendant la colonisation.

5. Procédé d'essai d'un agent pharmaceutique ou d'un traitement par radiothérapie comportant au moins les étapes suivantes :
- fabrication d'un tissu échantillon bioartificiel à vascularisation primaire présentant une matrice tissulaire biologique décellularisée à vascularisation primaire avec au moins une branche vasculaire, la matrice tissulaire étant colonisée par des cellules tumorales, souches, progénitrices ou hépatiques humaines et le tissu présentant un réseau capillaire et vasculaire,
- amenée de l'agent pharmaceutique dans un second milieu nutritif, qui est amené à l'au moins une branche vasculaire ou traitement par radiothérapie du tissu échantillon, et
- examen du tissu échantillon.

6. Procédé suivant la revendication 5, **caractérisé en ce qu'à** l'étape de la fabrication, la matrice tissulaire présente au moins une matrice d'une branche vasculaire artérielle et au moins une matrice d'une branche vasculaire veineuse.

7. Procédé suivant la revendication 5 ou 6, **caractérisé en ce qu'à** l'étape de la fabrication, l'au moins une branche vasculaire est reliée à un réseau capillaire intramural formé dans la matrice tissulaire.

8. Procédé suivant une des revendications 5 à 7, **caractérisé en ce qu'à** l'étape de la fabrication, le tissu échantillon est irrigué.

9. Procédé suivant une des revendications précédentes, **caractérisé en ce qu'à** l'étape de l'amenée de l'agent pharmaceutique, le second milieu nutritif amené à l'au moins une branche vasculaire est du sang, de préférence du sang autologue.

10. Procédé suivant une des revendications précédentes, **caractérisé en ce qu**'à l'étape de l'amenée de l'agent pharmaceutique, le second milieu nutritif est amené de façon pulsée.

11. Procédé suivant une des revendications précédentes, **caractérisé en ce que** le traitement par radiothérapie et l'amenée de l'agent pharmaceutique sont effectués simultanément et/ou de façon séparée dans le temps.

12. Procédé suivant une des revendications précédentes, **caractérisé en ce qu'à** l'étape de l'examen, le cycle cellulaire, la prolifération et les taux de synthèse du tissu échantillon sont examinés.

13. Procédé suivant une des revendications précédentes, **caractérisé en ce qu**'à l'étape de l'examen, des changements au niveau du taux de formation de nouveaux vaisseaux et des variations au niveau du profil d'expression de molécules d'adhésion sont examinés.

14. Procédé suivant une des revendications précédentes, **caractérisé en ce qu'à** l'étape de l'examen, les interactions au niveau ARN et protéique entre les cellules et les interactions entre les cellules et la matrice sont examinées.

15. Procédé suivant une des revendications précédentes, **caractérisé en ce qu**'à l'étape de l'examen, un métabolisme du tissu échantillon est examiné en contrôlant le milieu nutritif amené et évacué.
